# EUROPEAN PATENT APPLICATION

(11) **EP 4 124 864 A1**
(43) Date of publication of application: **01.02.2023**
(21) Application number: 21382687.8
(22) Date of filing: 26.07.2021
(51) Int. Cl.: G01N 33/564

(54) **IN VITRO METHOD FOR THE PROGNOSIS OF PATIENTS SUFFERING FROM SEPSIS**

(71) Applicant: Fundación Instituto de Estudios Ciencias de la Salud de Castilla y León (IECSCYL-IBSAL), 37007 Salamanca (ES); Universidad De Salamanca, 37008 Salamanca (ES); Fundación Para la Investigación Biomédica del Hospital Universitario de Getafe (FIB HUG), 28905 Getafe (Madrid) (ES); Consorcio Centro de Investigación Biomédica en Red, M.P. (CIBER), 28029 Madrid (ES)
(72) Inventor: FUENTES GARCÍA, Manuel, 37007 Salamanca (ES); LANDEIRA VIÑUELA, Alicia, 37007 Salamanca (ES); HERNÁNDEZ, Ángela Patricia, 37007 Salamanca (ES); JUANES VELASCO, Pablo, 37007 Salamanca (ES); LORENTE BALANZA, José Ángel, 28905 Getafe (Madrid) (ES); HERRERO HERNÁNDEZ, Raquel, 28905 Getafe (Madrid) (ES); FERRUELO ALONSO, Antonio, 28905 Getafe (Madrid) (ES)
(74) Representative: Hoffmann Eitle

(57) **Abstract**

The present invention refers to an *in vitro* method for the prognosis of patients suffering from sepsis, preferably COVID-19 patients. In a preferred embodiment, a bad prognosis means that the patient has an increased risk of developing Acute respiratory distress syndrome (ARDS).

## Description

### FIELD OF THE INVENTION

The present invention refers to the medical field. Particularly, the present invention refers to an *in vitro* method for the prognosis of patients suffering from sepsis, preferably COVID-19 patients. In a preferred embodiment, a bad prognosis means that the patient has an increased risk of developing Acute respiratory distress syndrome (ARDS).

### STATE OF THE ART

Coronavirus (CoV) are a family of single-stranded positive RNA viruses presenting highly diversity. This family is classified into three groups: Alphacoronavirus, Betacoronavirus and Gammacoronavirus. The first two infect mammals and the third are avian viruses. Currently, seven types of CoVs are known to be capable of infecting humans, two Alphacoronavirus (CoV-229E and CoV-NL63) and five Betacoronavirus (CoV-HKU1, CoV-OC43, SARS-CoV, MERS-CoV and SARS-CoV-2).

It is interesting to note that all CoVs have common characteristics. Structural proteins such as the spike glycoprotein (S) (responsible for virus-host cell interaction); envelope (E) and membrane (M) (accountable for the formation of the viral envelope); nucleocapsid (N) (in charge of forming a helical complex that interacts with the M protein during the virion assembly process). Also, non-structural proteins (involved in virus transcription and replication) and accessory proteins. During the process of CoV infection, the S protein is cleaved into two subunits (S1 and S2). In the S1 subunit is located the receptor binding domain (RBD) (responsible for the trimer organization of the S protein) and in the S2 subunit the fusion machinery.

While CoV-229E, CoV-NL63, CoV-OC43 and CoV-HKU1 exhibit low pathogenicity; SARS-CoV, MERS-CoV, and SARS-CoV-2 cause atypical pneumonias that can lead to acute respiratory distress syndrome (ARDS). As is well known, SARS-CoV 2 is currently considered a global health emergency. This CoV was discovered in December 2019 (COVID-19) due to the notification of an emerging outbreak in Wuhan (China). The main clinical features of patients of COVID-19 disease are very varied and can range from asymptomatic forms to severe atypical pneumonia.

As in any pathogen infection, the immune system plays a crucial role in the development and follow-up of COVID-19 disease. Both immune system strategies, the innate and adaptive response triggered by SARS-CoV 2 has attracted the attention of the scientific community. In general terms, innate immune system can be reflected as an inflammation response. This nonspecific response to environmental aggressions such as pathogens can also be induced by persistent tissue damage and the release of damage-associated patterns (DAMPs). These alterations in homeostasis are reflected in the imbalance of physiological crosstalk cascades such as coagulation, fibrinolysis, or the complement system.

With regards to the adaptive response, serum antibodies are essential components of adaptive immunity but are also involved in the pathogenesis of many autoimmune, allergies and oncopathologies. While it is well known about the control of host antibody production following pathogen or infectious exposure or vaccination; the induction of autoantibodies (AABs) in many other diseases remains still unclear in several aspects.

In genetically susceptible individuals, infection and other environmental factors have been described as factors which triggers immune responses by different mechanisms including cytokines productions and release, stimulation of toll-like receptors and other pattern recognition receptors, the release of self-antigens by damaged cells and tissues and /or molecular mimicry.

However, to date, it is still unknown about the spectrum of AAB responses and kinetics of AAB induction during acute infection and systemic inflammation.

Attending to COVID-19 disease, ARDS and sepsis are presented as one of the most serious clinical complications within patients. ARDS and sepsis are acute inflammatory conditions associated with high morbidity and mortality, often involving multiple organ failure. ARDS is caused by a wide variety of infectious or inflammatory stimulus to the lung that may occur by direct (ie. pneumonia) or indirect injury (ie. peritonitis). The pathology hallmarks of ARDS are diffuse alveolar damage manifested by disruption of alveolar capillary interface, as well as the accumulation of immune cells (innate and adaptive immune cells) and protein-rich exudates in the alveolar spaces. COVID-19 patients have elevated levels of cytokines and several inflammatory mediators (such as IL-6, TNF-α, IL-8, ...) in lung proximal fluids and peripheral blood. In SARS-CoV-2 infections caused local and systemic inflammatory response; however, in persistence of sepsis, there is a rapid shift towards an antiinflammatory immunosuppressive state, loss of dendritic cells, and low count of B cells and CD4+ lymphocytes; in contrast a cytokine storm with systemic consequences and the presence of AABs have been observed in COVID-19 patients.

Currently, there is an unmet medical need of finding reliable strategies focused on the prognosis of patients suffering from sepsis, preferably COVID-19 patients. Particularly, there is an unmet medical need of finding reliable strategies able to prognose whether a patient suffering from sepsis, preferably a COVID-19 patient, will develop ARDS.

The present invention is focused on solving this problem and a new strategy is herein provided for prognosing whether patients suffering from sepsis, preferably COVID-19, will develop ARDS.

### DESCRIPTION OF THE INVENTION

As cited above, the present invention refers to an *in vitro* method for the prognosis of patients suffering from sepsis, particularly for prognosing whether patients suffering from sepsis, preferably COVID-19 patients, will have a bad prognosis which, in the context of the present invention, means that the patient has an increased risk of developing ARDS.

Particularly, the inventors of the present invention have analysed the mechanism and time course of the rapid induction of AABs which occurs in patients suffering from sepsis, and particularly in patients suffering from COVID-19, who develop ARDS. So, in the context of the present invention, a multiplex array has been designed for simultaneous detection of acute phase components and AABs against a specific panel of autoantigens.

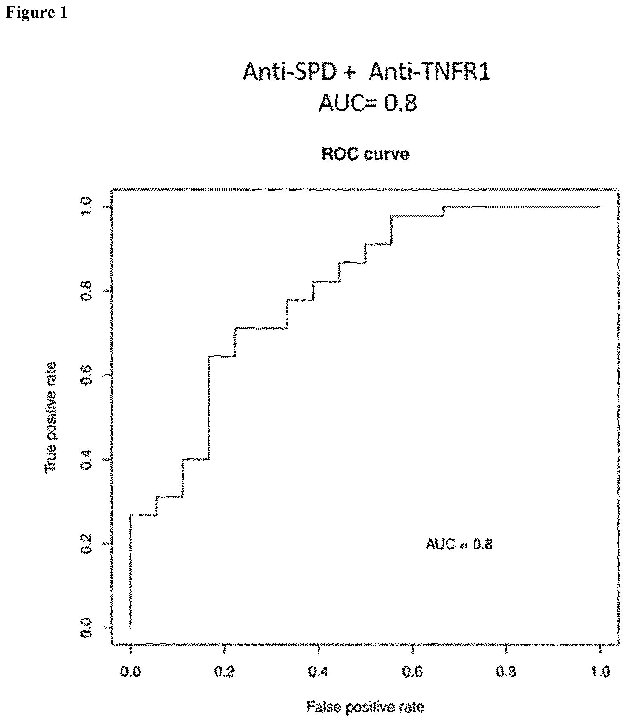

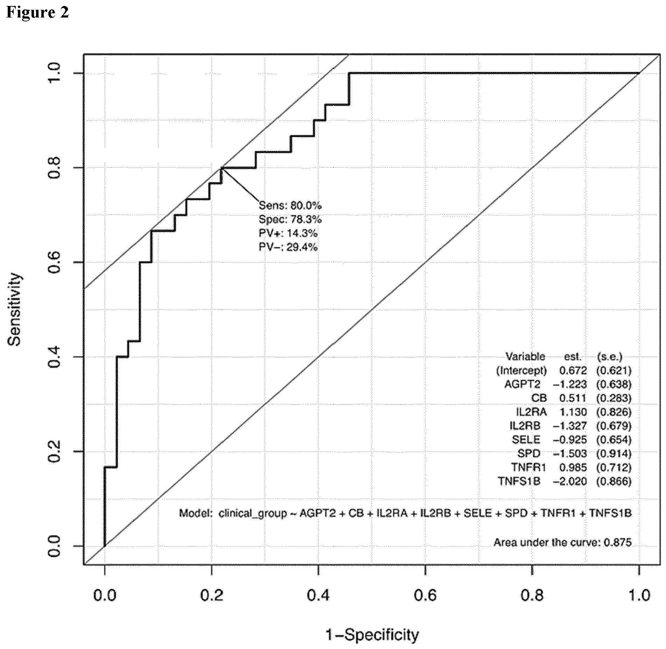

Particularly, **Figure 1** and **Figure 2** show ROC curves indicating an AUC value of 0.8 when AABs against [TNFR1 and SFTPD] were detected, and an AUC value of 0.875 when AABs against [AGPT2, CB, IL2RA, IL2RB, SELE, SFTPD, TNFR1 and TNFS1B] were detected. So, the identification of AABs against [TNFR1 and SFTPD] or [AGPT2, CB, IL2RA, IL2RB, SELE, SFTPD, TNFR1 and TNFS1B] is herein confirmed as a reliable strategy for prognosing whether patients suffering from sepsis, preferably COVID-19 patients, will have a bad prognosis which, in the context of the present invention, means that the patient has an increased risk of developing ARDS.

So, the first embodiment of the present invention refers to an *in vitro* method (hereinafter *"method of the invention"*) for the prognosis of patients suffering from sepsis which comprises assessing the presence of AABs against [TNFR1 and SFTPD] in a biological sample obtained from the patient, wherein the presence of AABs against [TNFR and SFTPD] is an indication that the patient suffering from sepsis has a bad prognosis.

In a preferred embodiment, the present invention comprises assessing the presence of AABs against [AGPT2, CB, IL2RA, IL2RB, SELE, SFTPD, TNFR1 and TNFS1B] in a biological sample obtained from the patient, wherein the presence of AABs against [AGPT2, CB, IL2RA, IL2RB, SELE, SFTPD, TNFR1 and TNFS1B] is an indication that the patient suffering from sepsis has a bad prognosis.

In a preferred embodiment, the presence of AABs against [TNFR1 and SFTPD] or [AGPT2, CB, IL2RA, IL2RB, SELE, SFTPD, TNFR1 and TNFS1B] is an indication that the patient suffering from sepsis will develop ARDS.

In a preferred embodiment, the method of the invention further comprises assessing the presence of autoantibodies against [TNFRSF6B, MUC1, MOK and CXCL8] and/or [IL2RB, SFTPD, TNFRSF1B and AGPT2] in a biological sample obtained from the patient, wherein the presence of autoantibodies against [TNFRSF6B, MUC1, MOK and CXCL8] and/or [IL2RB, SFTPD, TNFRSF1B and AGPT2] is an indication that the patient will develop ARDS with high severity. So, the assessment of autoantibodies against [TNFRSF6B, MUC1, MOK and CXCL8] and/or [IL2RB, SFTPD, TNFRSF1B and AGPT2] can be used for stratifying or differentiating patients who will develop ARDS with high (or higher) severity from other patients who will develop ARDS with low (or lower) severity.

The second embodiment of the present invention refers to the *in vitro* use of [TNFR and SFTPD] or of AABs against [TNFR and SFTPD] for the prognosis of patients suffering from sepsis.

In a preferred embodiment, the present invention refers to the *in vitro* use of [AGPT2, CB, IL2RA, IL2RB, SELE, SFTPD, TNFR1 and TNFS1B] or of AABs against [AGPT2, CB, IL2RA, IL2RB, SELE, SFTPD, TNFR1 and TNFS1B] for the prognosis of patients suffering from sepsis.

In a preferred embodiment, [TNFR1 and SFTPD] or [AGPT2, CB, IL2RA, IL2RB, SELE, SFTPD, TNFR1 and TNFS1B], or AABs against [TNFR1 and SFTPD] or [AGPT2, CB, IL2RA, IL2RB, SELE, SFTPD, TNFR1 and TNFS1B], are used for prognosing whether a patient suffering from sepsis will develop ARDS.

In a preferred embodiment, [TNFRSF6B, MUC1, MOK and CXCL8] and/or [IL2RB, SFTPD, TNFRSF1B and AGPT2], or AABs against [TNFRSF6B, MUC1, MOK and CXCL8] and/or [IL2RB, SFTPD, TNFRSF1B and AGPT2] are used for prognosing whether a patient will develop ARDS with high (or higher) severity. So, the assessment of autoantibodies against [TNFRSF6B, MUC1, MOK and CXCL8] and/or [IL2RB, SFTPD, TNFRSF1B and AGPT2] can be used for stratifying or differentiating patients who will develop ARDS with high (or higher) severity from other patients who will develop ARDS with low (or lower) severity.

The third embodiment of the present invention refers to a kit consisting of reagents for determining the presence of autoantibodies against [TNFR and SFTPD] or [AGPT2, CB, IL2RA, IL2RB, SELE, SFTPD, TNFR1 and TNFS1B] and, optionally, against [TNFRSF6B, MUC1, MOK and CXCL8] and/or [IL2RB, SFTPD, TNFRSF1B and AGPT2].

In a preferred embodiment, the kit is used for the prognosis of patients suffering from sepsis.

In a preferred embodiment, the kit is used for prognosing whether a patient suffering from sepsis will develop ARDS.

In a preferred embodiment, the kit is used for stratifying or differentiating patients who will develop ARDS with high (or higher) severity from other patients who will develop ARDS with low (or lower) severity.

In a preferred embodiment, the patient suffering from sepsis is a COVID-19 patient.

In a preferred embodiment, the patient suffering from sepsis is a patient infected with coronavirus SARS-CoV-2.

In a preferred embodiment, the biological sample is blood, serum or plasma.

In a preferred embodiment, the method of the invention comprises detecting and/or quantifying autoantibodies against the autoantigens [TNFR and SFTPD] or [AGPT2, CB, IL2RA, IL2RB, SELE, SFTPD, TNFR1 and TNFS1B], and optionally against autoantigens [TNFRSF6B, MUC1, MOK and CXCL8] and/or [IL2RB, SFTPD, TNFRSF1B and AGPT2], preferably by using Self-assembled protein Arrays (SAPA), wherein purified template DNA of the autoantigens (including the protein of interest and a tag molecule) is printed onto a slide together with an antibody that recognizes the specific tag. When the biological sample is added, the transcription and translation are initiated, and the expressed protein is captured by the anti-tag antibody.

On the other hand, the present invention also refers to the topics described below.

A method for detecting autoantibodies against [TNFR and SFTPD] or [AGPT2, CB, IL2RA, IL2RB, SELE, SFTPD, TNFR1 and TNFS1B], and optionally against [TNFRSF6B, MUC1, MOK and CXCL8] and/or [IL2RB, SFTPD, TNFRSF1B and AGPT2], in a test sample from a human patient suffering from sepsis, preferably a COVID-19 patient, at risk of developing ARDS, the method comprising:
a) Printing purified cDNA encoding the full-length sequence for the autoantigens [TNFR and SFTPD] or [AGPT2, CB, IL2RA, IL2RB, SELE, SFTPD, TNFR1 and TNFS1B], and optionally [TNFRSF6B, MUC1, MOK and CXCL8] and/or [IL2RB, SFTPD, TNFRSF1B and AGPT2], onto a slide together with an antibody that recognizes the specific tag, and
b) Capturing the expressed protein by the anti-tag antibody when the biological sample is added.

In a preferred embodiment, the present invention is a computer-implemented invention, wherein a processing unit (hardware) and a software are configured to:
- Receive information/data regarding the presence of autoantibodies against [TNFR and SFTPD] or [AGPT2, CB, IL2RA, IL2RB, SELE, SFTPD, TNFR1 and TNFS1B] and optionally against [TNFRSF6B, MUC1, MOK and CXCL8] and/or [IL2RB, SFTPD, TNFRSF1B and AGPT2],
- Process the information/data received for finding substantial variations or deviations with respect to the presence or absence of autoantibodies against [TNFR and SFTPD] or [AGPT2, CB, IL2RA, IL2RB, SELE, SFTPD, TNFR1 and TNFS1B] and optionally against [TNFRSF6B, MUC1, MOK and CXCL8] and/or [IL2RB, SFTPD, TNFRSF1B and AGPT2], and
- Provide an output through a terminal display of the variation or deviation detected, wherein the presence of autoantibodies against [TNFR and SFTPD] or [AGPT2, CB, IL2RA, IL2RB, SELE, SFTPD, TNFR1 and TNFS1B] is an indication that the patient suffering from sepsis has a bad prognosis and could develop ARDS, and wherein the presence of autoantibodies against [TNFRSF6B, MUC1, MOK and CXCL8] and/or [IL2RB, SFTPD, TNFRSF1B and AGPT2] is an indication that the patient will develop ARDS with high severity.

The present invention also refers to a computer program or a computer-readable media containing means for carrying out the method of the invention.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- By "consisting of" it is meant including, and limited to, whatever follows the phrase "consisting of'. Thus, the phrase "consisting of" indicates that the listed elements are required or mandatory, and that no other elements may be present.

### Description of the figures

**Figure 1****.** It shows a ROC curve indicating an AUC value of 0.8 when AABs were detected against [TNFR1 and SFTPD]. SPD = SFTPD (pulmonary surfactant protein D).
**Figure 2****.** It shows a ROC curve indicating an AUC value of 0.875 when AABs were detected against [AGPT2, CB, IL2RA, IL2RB, SELE, SFTPD, TNFR1 and TNFS1B]. SPD = SFTPD (pulmonary surfactant protein D).

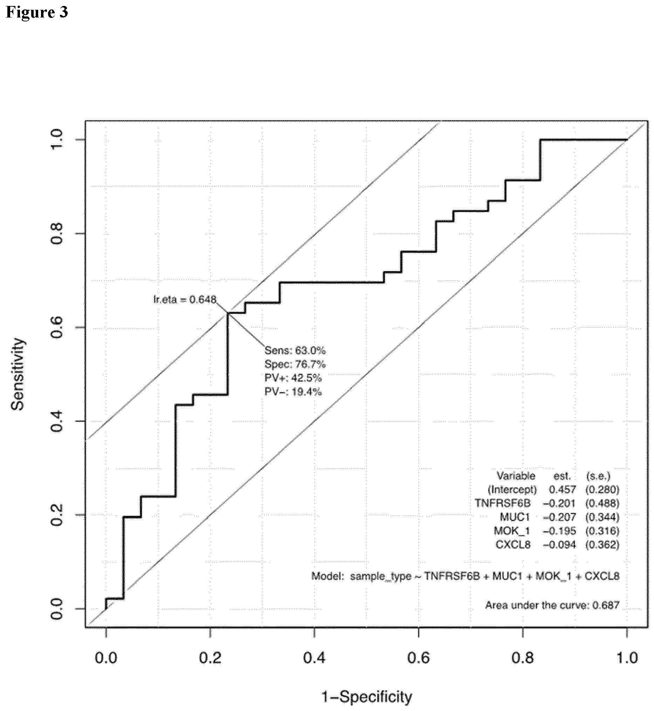

**Figure 3****.** Predictive value of AAB profile analysis at COVID-19 ARDS prognosis. Protein microarray profile of AAB for [TNFRSF6B, MUC1, MOK_1 and CXCL8] was employed to generate a Random Forest (RF) classification model of ARDS prognosis. RF performance was evaluated by calculating AUC and ROC curves when classifying individuals at cohort 2 with mild symptoms (no ICU) and patients admitted to ICU.

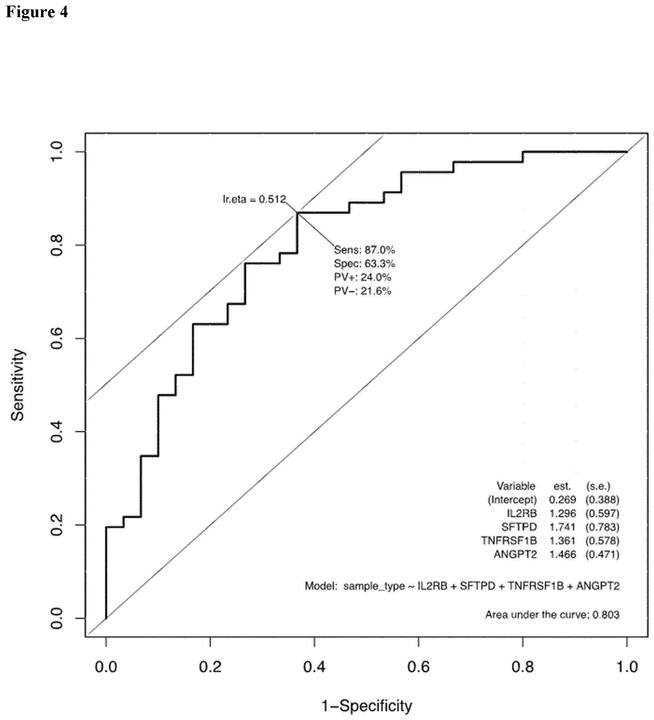

**Figure 4****.** Predictive value of AAB profile analysis at COVID-19 ARDS prognosis. Protein microarray profile of AAB for [IL2RB, SFTPD, TNFRSF1B and ANGPT2] was employed to generate a Random Forest (RF) classification model of ARDS prognosis. RF performance was evaluated by calculating AUC and ROC curves when classifying individuals at cohort 2 with mild symptoms (no ICU) and patients admitted to ICU.

### Detailed description of the invention

The present invention is illustrated by means of the examples set below without the intention of limiting its scope of protection.

### Example 1. Material and methods

### Example 1.1. Materials

All reagents were of analytical grade and were used as received without further purification. Sodium acetate (AcONa), isopropyl alcohol, ethanol 96 %, 3-(2-Aminoethylamino)-propyldimethoxymethylsilane (MANAE) (≥95.0 %) (Cat. no. 06667), BS3 (bis(sulfosuccinimidyl)suberate) (Cat. no. 21580), dimethyl sulfoxide (DMSO), glycerol 85 %, bovine serum albumin (BSA), Tween^{™} 20, Hybriwell sealing system (Cat. no GBL612105), Lysogeny broth (LB) medium, Grace Bio-Labs ProPlate^{®} microarray system (Cat. no GBL246858), Grace Bio-Labs ProPlate^{®} clips for microarray systems (Cat. no. GBL204838) (Sigma-Aldrich, St. Louis/MO, USA); SuperBlock^{™} Blocking Buffer (TBS) (Cat. no. 37535), Pierce^{™} BCA Protein Assay Kit. Ref. (Cat. no. 23225), EZ-Link NHS-PEG4 Biotin. (Cat. no. 21330), Blocker^{™} BSA (10 %) in PBS (Cat. no. 37525), Quant-it Pico Green dsDNA Assay Kit (Cat. no. P7589), Microscope slides pre-cleaned and polished with ground edges (76 x 26 mm), Mseries lifterlip y LifterslipTM coverslips, Coronavirus Ig Total Human 11-Plex ProcartaPlex^{™} Panel (Cat. no. EPX110-16000-901) (Thermo Scientific, Rockford/IL, USA); Microarray-Specific 384-well Microplates, JetStar^{™}, Optimum Microarray Printing Buffer C (ArrayJet, Roslin, UK); Corning^{®} 96-well Black Flat Bottom Polystyrene Not Treated Microplate, 25 per Bag, without Lid, Nonsterile (Cat. no. 3915) (Corning, Somerville, Massachusetts, USA); Cytiva Amersham^{™} Streptavidin-Fluor Cy3 (Cat. no. PA43001) (GE-HEALTCARE, Little Chalfont, Buckinghamshire, UK); MAGPIX^{®} Drive Fluid, 4 pack (Cat. no. MPXDF-4PK) (Merck KGaA, Darmstadt, Germany); TnT^{®} Coupled Reticulocyte Lysate System kit (Cat. no. L4610), Pure Yield plasmid miniprep system (Cat. no. A1222) (Promega, Madison/WI, USA); TSA Individual Cyanine 3 Tyramid Reagent Pack (Cat. no. SAT704B001EA) (PerkinElmer, Waltham/MA, USA).

### Example 1.2. Equipment and software

ArrayJet^{®} Printer Marathon v1.4, JetSpyder^{™} 12 samples, JetStar^{™} (ArrayJet, Roslin, UK); Scanner SensoSpot Fluorescence (Miltenyi Imaging GmbH, Radolfzell, Germany); Orbital shaker (FALC Instruments S.r.l.; Treviglio, Italy); Fisherbrand^{™} Microplate Vortex Mixers (Fisherbrand^{™}, EEUU); T100 Thermal Cycler (Biorad, Hercules/CA, USA); Magnetic 96-Well Separator, Digital Dry Block Heater (Thermo Scientific, Rockford/IL, USA); GenePix^{®} Pro Microarray Analysis Software (Molecular Devices, San Jose/CA, USA); R statistics software (R Foundation for Statistical Computing, Vienna, Austria. http://www.R-project.org/); MAGPIX^{®} System of xMAP^{®} instruments and xPONENT^{®} Software (Luminex Corporation, Austin, Texas, USA).

### Example 1.3. Patients and Samples

### Example 1.3.1. Cohort 1

Plasma samples from 20 patients diagnosed of COVID-19 by PCR and 10 healthy donors (COVID-19 negative) were collected in the Hospital University of Salamanca (HUS, Salamanca, Spain) and deposited in the Biobank Spanish National DNA (Banco Nacional de ADN, University of Salamanca). Each individual gave informed consent prior to entering the study and was subsequently approved by the HUS ethics committee. The most relevant clinical and laboratory information are summarized in **Table 1.**

**Table 1**

| **Characteristics** | | **ARDS Positive (n=20)** | **ARDS Negative (n=10)** | **Total Cases (n=30)** |
|---|---|---|---|---|
| **Age (Years)** | ***Minimum*** | 55 | 18 | 18 |
| | ***Maximum*** | 92 | 65 | 92 |
| | ***Median*** | 70 | 39 | 59 |
| **Gender** | ***Female*** | 10 (50 %) | 3 (30%) | 13 (43.3%) |
| | ***Male*** | 10 (50 %) | 7 (70 %) | 17 (56.7%) |

| **SARS-Cov2 Serological test** | | | | |
|---|---|---|---|---|
| **Positive** | ***Ig G*** | 16 (80 %) | - | 16 (53.3 %) |
| | ***Ig M*** | 10 (50 %) | - | 10 (33.3 %) |
| | ***Both Ig*** | 6 (30 %) | - | 6 (20%) |
| | ***Only Ig G*** | 10 (50 %) | - | 10 (33%) |
| | ***Only Ig M*** | 4 (20 %) | - | 4 (13.3 %) |
| **SARS-Cov2 PCR test** | | 19 (95 %) | - | 19 (63.3 %) |

### Example 1.3.2. Cohort 2

Plasma samples from 76 patients diagnosed of COVID-19 by RT- PCR were collected in the Hospital University Miguel Servet (HUMS, Zaragoza, Spain). The most relevant biological-clinical information is summarized in **Table 2.**

**Table 2**

| **Characteristics** | **ARDS Positive (n=30)** | **ARDS Negative (n=46)** | **Total cases (n=76)** |
|---|---|---|---|
| **Age (years)** | | | |
| **Min** | 42 | 31 | 31 |
| **Max** | 89 | 86 | 89 |
| **Mean** | 70 | 60 | 65 |
| **Gender** | | | |
| Female | 12 (40%) | 23 (50%) | 35(46%) |
| Male | 18 (60%) | 23 (50%) | 41(54%) |

| **COVID-19 severity** | | | |
|---|---|---|---|
| Non hospitalized | - | 16 (34.8%) | 16 (21%) |
| Hospitalized | - | 30 (65.2%) | 30 (39.5%) |
| ICU | 10 (33.3%) | - | 10 (13.2%) |
| Exitus | 20 (66.6%) | - | 20 (26.3%) |

### Example 1.4. AAB profiling by Self-assembled protein Arrays (SAPA)

### Example 1.4.1. SAPA design and content for serum AAB profiling

SAPA was built with 30 cDNAs encoding ARDS AABs in triplicate and positive (Cy3, MasterMix) and negative (GST-antibody, PBS, bovine serum albumin (BSA), Bis-(sulfosuccinimidyl) suberate (BS3), clean buffer, and printing buffer) controls. The design and distribution of the cDNAs on the arrays was as follows: 6 subarrays, each subarray with 144 spots, deposited on a chemically activated surface with ArrayJet Printer Marathon v1.4.

In order to perform the cDNA processing and plasmid sequencing for these SAPA we followed the protocols of LaBaer's lab. *Escherichia coli* bearing a total of 30 sequence-verified full-length human genes in pANT7_cGST were obtained from the Center for Personalized Diagnostics at the Arizona State University and are publicly available (www.dnasu.org), compatible with *in vitro* transcription/translation (IVTT) method. Bacteria were grown for 24 h at 37 °C in 100 mL of Luria-Bertani medium supplemented with ampicillin. The cells were pelleted, and their plasmid DNA was purified using the Pure Yield plasmid miniprep system according to the manufacturer's instructions.

Purified cDNAs were precipitated by the addition of 0.8× volumes of isopropanol and centrifugation at 4000g for 30 min at RT. Precipitated cDNAs were then washed with 80 % ethanol and allowed to air-dry. cDNA (15 µg) of each precipitated plasmid were dissolved in 15 µL of MasterMix solution containing 33.3 mg/mL BSA, 2.5 mg/mL rabbit polyclonal anti-GST antibody and 2 mM BS3 and transferred to a 384-plate previously charged with 15 µL of glycerol 47 % (v/v).

### Example 1.4.2. SAPA performance

### Example 1.4.2.1. Quality Control (QC)

In all the SAPA assays, to verify the correct arrangement of the cDNA on the microarray surface, microarrays were blocked, and cDNA staining was carried out incubating each microarray with 200 µL of picogreen solution diluted 1:600 (v/v). In addition, to observe *in situ* protein expression, arrays were blocked and incubated with IVTT system kit (TnT^{®} Coupled Reticulocyte Lysate System kit, Promega, Cat. no. L4610).

### Example 1.4.2.2. Serum screening

For plasma serum screening, microarrays were blocked, washed with distilled water and dried with compressed nitrogen air. Proteins were then expressed using the protocol for the IVTT system. The master mix for this IVTT system was prepared with 200 µL of reticulocyte lysate containing 16 µL of TNT buffer, 8 µL of T7 polymerase, 4 µL of -Met, 4 µL of -Leu or -Cys and 168 µL of DEPC water and proceeding following the manufacturer's instructions. The IVTT system was incubated on the microarrays, using HybriWell gaskets. The microarrays were incubated for 90 min at 30 °C and 30 min at 15 °C for protein expression and capture by the polyclonal anti-GST antibody. The HybriWell gaskets were then removed and the arrays were washed with MixMilk (PBS, 0.5 g milk powder and 0.2 % tween20) 1 h on an orbital shaker. The microarrays were washed with distilled water and both cohorts were incubated in each subarray (1:100 (v/v) in MixMilk at 4 °C in rotation overnight. The next day, the microarrays were washed three times with PBST 0.05 %. First, microarrays were then incubated with HRP-linked anti-human IgG for 30 min at a dilution of 1:200 and then washed again three times with PBST 0.05 %. Secondly, microarrays were incubated with 200 µL/microarray of tyramide signal amplification reagent for 5 min at RT. Microarrays were then washed three times with 0.05 % PBST, once with distilled water and dried by centrifugation.

### Example 1.4.2.3. Image acquisition

As described above, array images were obtained by Scanner SensoSpot Fluorescence. The TIFF images generated by array scanning were analyzed using GenePix Pro 6.0. software. Parameters were set to quantify light intensity values at Cy3 (λ= 532 nm).

### Example 1.5. Bioinformatics Analysis

### Example 1.5.1. AAB microarray data pre-processing and QC

The fluorescence signal retrieved from images processed in the previous section was corrected by subtracting background signal and then transformed into Z score. Overall raw fluorescence and log₂ (Z score) density distribution were compared to validate signal correction at each microarray employed. Principal Component Analysis (PCA) was performed to discard any microarray-wise batch effect. Data processing and analysis were performed in R environment.

### Example 1.5.2. Biostatistics and Data visualization

Volcano plots illustrate the statistical significance of Z score ratio changes at any two defined conditions. Z ratio is calculated by subtracting the mean Z score in condition A and mean Z score in condition B and then dividing by the overall standard deviation of Z score in conditions A and B. Volcano plot Y-axis represent the statistical significance of Z score mean difference in condition A and B -Wilcoxon Rank Sum test, - log₂ (p-value). Canonical biplot is a visualization technique extensively applied to interpret Principal Component Analysis (PCA). The biplots presented in this work draw both observation and variables -patient samples and microarray proteins respectively- as dots and directed vectors. The vector size and direction indicate the discriminatory power of protein variables at the first two Principal Components. Importantly, the direction of vector variables at biplot can also reveal correlations between sets of protein variables and therefore, corroborate the trends observed in Volcano plots.

All the dendograms depicted in the heat maps presented in this work were generated applying the Euclidean distance and Complete-linkage clustering method. Protein microarray profile of AAB for IL2RB, SFTPD, TNFRSF1B and ANGPT2 was employed to generate a Random Forest (RF) classification model of ARDS prognosis. RF performance was evaluated by calculating AUC and ROC curves when classifying individuals at cohort 2 with mild symptoms (no ICU) and patients admitted to ICU. RF and ROC curves were generated using randomForest and EPI R packages -ntry =2, ntree=500-. The plots presented in this work were generated using *ggplot2, ggpubr, ggbiplot, Epi, ComplexHeatmap* and *pathview* R package.

In addition, AAB protein microarray profile was employed to generate a series of Random Forest (RF) classification models of ARDS prognosis. RF performance was evaluated calculating AUC and ROC curves when classifying individuals with mild symptoms (no ICU) and patients admitted to ICU. RF and ROC curves were generated using randomForest and EPI R packages -ntry =2, ntree=500-.

### Example 2. Results

### Example 2.1. SARS-CoV-2 Antigen profiling

Antigenic multiplex assay for other coronaviruses indicates that 87 % has S1 protein antibodies for CoV-229E, 97 % for CoV-HKU1, 90 % for CoV-OC43, 97 % for CoV-NL63 and 33 % for SARS-CoV.

When analysing these data according to ARDS diagnosis (positive or negative) we obtained that for: i) ARDS negative: 90 % of samples have antibodies for CoV-229E, CoV-HKU1 and CoV-NL63 and 100 % have antibodies for CoV-OC43, 97 % for and SARS-CoV. ii) ARDS positive: 85 % of samples have antibodies for CoV-229E and CoV-OC43, 100 % have antibodies for CoV-HKU1 and CoV-NL63 and 50% have antibodies for SARS-CoV protein **(Table 3** and **Table 4).**

**Table 3**

| Antigenic Multiplex Assay | | | | | Serologic test | | | | PCR test |
|---|---|---|---|---|---|---|---|---|---|
| Spike Trimer | S1 Protein | RBD | Nucleocapsid | | IgG | IgM | IgG and IgM | | |
| 45 % | 75 % | 30 % | 15 % | | 80 % | 50 % | 12 % | | 95 % |

**Table 4**

| Type of Coronavirus | Antibody detected | Protein target |
|---|---|---|
| CoV-229E | Ig Total | S1 |
| CoV-HKU1 | Ig Total | S1 |
| CoV-OC43 | Ig Total | S1 |
| CoV-NL63 | Ig Total | S1 |
| SARS S1 | Ig Total | S1 |
| SARS-CoV2 | Ig Total | RBD |
| SARS-CoV2 | Ig Total | Nucleocapsid |
| SARS-CoV2 | Ig Total | Spike (Trimer) |
| SARS-CoV2 | Ig Total | S1 Protein |

This same assay for SARS-CoV-2 indicates that the protein with the highest antigenic capacity is the S1 protein, present in 75 % of the COVID-19 patients. This is followed by the trimeric form of the S1 protein in 45 % of samples, RBD in 30% of samples and nucleocapsid in 15 % of samples. 80 % of COVID-19 patients and with ARDS have IgG antibodies for protein N and 50 % presented Ig M. Both immunoglobulins were present in 30% COVID-19 patients.

As well, when we have a Wilcoxon mean for samples positive or negative COVID-19, we do nor observe significant differences between present of coronaviruses and ARDS diagnostic, although there are significant differences between present of proteins relation with SARS-CoV-2 (Spike, S1 protein, RBD and Nucleocapsid).

### Example 2.2. AAB profile in ARDS patients

Considering an exploratory analysis of the AAB profile in the cohort 1 (n=30), differences can be found between ARDS positive and ARDS negative (control) patients. A total of 30 AABs **(Table 5)** corresponding to cytokines, tissue damage, extracellular matrix proteins, lung tissue proteins and antigenic proteins associated with other pathologies associated with lung tissue damage, among others, will be studied for both groups of patients.

**Table 5**

| Official Symbol provided by HGNC | Official Full Name provided by HGNC | Gene ID NCBI | Uniprot ID |
|---|---|---|---|
| ANGPT2 | angiopoietin 2 | 285 | 015123 |
| CALCA | calcitonin related polypeptide alpha | 796 | P01258 |
| CAV1 | caveolin 1 | 857 | Q03135 |
| CAV2_1 | caveolin 2 | 858 | P51636 |
| CAV2_2 | caveolin 2 | 858 | P51636 |
| CPA4 | carboxypeptidase A4 | 51200 | Q9UI42 |
| FGF7 | fibroblast growth factor 7 | 2252 | P21781 |
| HGF | hepatocyte growth factor | 3082 | P08581 |
| HMGB1 | high mobility group box 1 | 3146 | P09429 |
| ICAM1 | intercellular adhesion molecule 1 | 3383 | P05362 |
| IL10 | interleukin 10 | 3586 | P22301 |
| IL2RA | interleukin 2 receptor subunit alpha | 3559 | P01589 |
| IL2RB | interleukin 2 receptor subunit beta | 3560 | P14784 |
| IL2RG | interleukin 2 receptor subunit gamma | 3561 | P31785 |
| IL6 | interleukin 6 | 3569 | P05231 |
| CXCL8 | C-X-C motif chemokine ligand 8 | 3576 | P10145 |
| MUC1 | mucin 1, cell surface associated | 4582 | P15941 |
| SERPINE1 | serpin family E member 1 | 5054 | P05121 |
| PROC | protein C, inactivator of coagulation factors Va and VIIIa | 5624 | P04070 |
| PTHLH | parathyroid hormone like hormone | 5744 | P12272 |
| MOK_1 | MOK protein kinase | 5891 | Q9UQ07 |
| MOK_2 | MOK protein kinase | 5891 | Q9UQ07 |
| SELE | selectin E | 6401 | P16581 |
| SELL | selectin L | 6402 | P14151 |
| SFTPB | surfactant protein B | 6439 | P07988 |
| SFTPD = SPD | surfactant protein D | 6441 | P35247 |
| TNFRSF1A | TNF receptor superfamily member 1A | 7132 | P19438 |
| TNFRSF1B | TNF receptor superfamily member 1B | 7133 | P20333 |
| TNFRSF6B | TNF receptor superfamily member 6B | 8771 | O95407 |
| VWA1 | von Willebrand factor A domain containing 1 | 64856 | Q6PCB0 |

Of these AABs, there are three (ANGPT2, SELE and CAV2) that are expressed in ARDS-positive patients with a highly significant difference (Wilcoxon test p-value < 0.05) over patients without ARDS. Otherwise occurs in control patients, where a highly significant difference (Wilcoxon test p-value < 0.05) is found in MOK expression compared to ARDS patients.

Once the most significant differences between patient groups have been identified, a clear correlation is observed between the intensity of AAB expression in ARDS-positive patients and age. The older the age of the patients, the higher the intensity of AAB expression is found. So, the distribution of proteins is mainly by age group. A high expression of AABs can be observed especially in patients between 60 and 80 years.

In this age range, we found proteins with high expression such as ANGPT2, CAV2, FGF7 and PROC. In contrast, in patients older than 80 years, a highly significant difference (pWilcoxon test p-value < 0.05) is observed in the expression of MUC1, TNFRSF6B, CXCL8 and MOK.

### Example 2.3. Validation of specific combination of autoantigens.

After the analysis of all the autoantigens described above, different ROC curves where generated in order to identify the combination of autoantigens offering the best statistical results in terms of AUC, sensitivity and specificity.

Particularly, such as it can be seen in **Figure 1** and **Figure 2****,** two signatures offered a significantly high AUC for the prognosis of patients suffering from sepsis, particularly for prognosing whether patients suffering from sepsis, preferably COVID-19 patients, will have a bad prognosis which, in the context of the present invention, means that the patient has an increased risk of developing ARDS.

Specifically, **Figure 1** and **Figure 2** show ROC curves indicating an AUC value of 0.8 when AABs against [TNFR1 and SFTPD] were detected, and an AUC value of 0.875 when AABs against [AGPT2, CB, IL2RA, IL2RB, SELE, SFTPD, TNFR1 and TNFS1B] were detected. So, the identification of AABs against [TNFR1 and SFTPD] or [AGPT2, CB, IL2RA, IL2RB, SELE, SFTPD, TNFR1 and TNFS1B] is herein confirmed as a reliable strategy for prognosing whether patients suffering from sepsis, preferably COVID-19 patients, will have a bad prognosis which, in the context of the present invention, means that the patient has an increased risk of developing ARDS.

### Example 2.4. Evaluation of AAB profiling across COVID-19 severity

Once we have observed the AABs profile, we investigated if this profile can be correlated with the grade of severity of the disease. Therefore, we evaluated the AAB profile in a larger cohort of COVID-19 positive patients (n=76), which includes clinical-biological characteristics divided in four differentiated groups of patients depending on the severity of the pathology.

In reference to severity, in order to compare a greater number of AABs expressed in patients with lower and higher severity, two differentiated groups of patients were made. On the one hand, 'No ICU', which includes non-hospitalized and hospitalized patients. On the other hand, 'ICU', which includes ICU and exitus individuals.

Between these two groups, there are significant differences (Wilcoxon test p-value < 0. 05) where the expression of TNFRSF6B (tumor necrosis factor), MUC1 (prognostic tumor marker), MOK and CXCL8 (involved in inflammation) is higher in the 'No ICU' group; while the expression of FGF7 (involved in cell growth and tissue repair), TNFRSF1B (tumor necrosis factor), CPA4 (involved in proteolysis), ANGPT2 (involved in vascular remodelling), PROC (involved in blood clotting), CAV1 (cell cycle regulation), SELE (involved in inflammation), IL2RA and IL2RB (involved in extracellular proteolysis) is higher in the 'ICU' group.

Several AABs such as MUC1 or TNFRSF6B, among others, are observed with a higher expression in patients with a more severe pathology. In addition, as previously indicated in the cohort 1, a higher signal intensity is observed in older patients. On the contrary, a high intensity of FGF7 and ANGPT2 expression, among other AABs, is also observed in patients with less severity.

In addition, AAB proteins showing largest Z ratios at AAB profiling, were used to define a series of Random Forest (RF) models to classify ARDS COVID-19 patient severity. The performance of RF models at classifying patients admitted to ICU was assessed by calculating the Area Under the Curve (AUC). The combinatorial use of [TNFRSF6B, MUC1, MOK and CXCL8] shows an AUC=0.687, with a 63% of sensitivity and a 76.7% of specificity **(****Figure 3****).** However, the combinatorial use of AAB proteins significantly decreased at patients admitted to ICU [IL2RB, SFTPD, TNFRSF1B and AGPT2] returned the RF model with best performance (AUC=0.8) **(****Figure 4****),** with an 87% of sensitivity and a 63.3% of specificity.

## Claims

1. *In vitro* method for the prognosis of patients suffering from sepsis which comprises assessing the presence of autoantibodies against [TNFR1 and SFTPD] in a biological sample obtained from the patient, wherein the presence of autoantibodies against [TNFR and SFTPD] is an indication that the patient suffering from sepsis has a bad prognosis.

2. *In vitro* method, according to claim 1, which comprises assessing the presence of autoantibodies against [AGPT2, CB, IL2RA, IL2RB, SELE, SFTPD, TNFR1 and TNFS1B] in a biological sample obtained from the patient, wherein the presence of autoantibodies against [AGPT2, CB, IL2RA, IL2RB, SELE, SFTPD, TNFR1 and TNFS1B] is an indication that the patient suffering from sepsis has a bad prognosis.

3. *In vitro* method, according to any of the claims 1 or 2, wherein the presence of autoantibodies against [TNFR and SFTPD] or [AGPT2, CB, IL2RA, IL2RB, SELE, SFTPD, TNFR1 and TNFS1B] is an indication that the patient suffering from sepsis will develop Acute respiratory distress syndrome (ARDS).

4. *In vitro* method, according to any of the previous claims, wherein the patient suffering from sepsis is a COVID-19 patient, preferably a patient infected with coronavirus SARS-CoV-2.

5. *In vitro* method, according to any of the previous claims, which further comprises assessing the presence of autoantibodies against [TNFRSF6B, MUC1, MOK and CXCL8] and/or [IL2RB, SFTPD, TNFRSF1B and AGPT2] in a biological sample obtained from the patient, wherein the presence of autoantibodies against [TNFRSF6B, MUC1, MOK and CXCL8] and/or [IL2RB, SFTPD, TNFRSF1B and AGPT2] is an indication that the patient will develop ARDS with high severity.

6. *In vitro* method, according to any of the previous claims, wherein the biological sample is blood, serum or plasma.

7. *In vitro* use of [TNFR and SFTPD], or of autoantibodies against [TNFR and SFTPD], for the prognosis of patients suffering from sepsis.

8. *In vitro* use, according to claim 7, of [AGPT2, CB, IL2RA, IL2RB, SELE, SFTPD, TNFR1 and TNFS1B], or of or of autoantibodies against [AGPT2, CB, IL2RA, IL2RB, SELE, SFTPD, TNFR1 and TNFS1B], for the prognosis of patients suffering from sepsis.

9. *In vitro* use, according to any of the claims 7 or 8, for prognosing whether a patient suffering from sepsis will develop ARDS.

10. *In vitro* use, according to any of the claims 7 to 9, wherein the patient suffering from sepsis is a COVID-19 patient, preferably a patient infected with coronavirus SARS-CoV-2.

11. *In vitro* use, according to any of the claims 7 to 10, of [TNFRSF6B, MUC1, MOK and CXCL8] and/or [IL2RB, SFTPD, TNFRSF1B and AGPT2], or of autoantibodies against [TNFRSF6B, MUC1, MOK and CXCL8] and/or [IL2RB, SFTPD, TNFRSF1B and AGPT2] for prognosing whether the patient will develop ARDS with high severity.

12. Kit consisting of reagents for determining the presence of autoantibodies against:
a. [TNFR and SFTPD] or [AGPT2, CB, IL2RA, IL2RB, SELE, SFTPD, TNFR1 and TNFS1B] and, optionally,
b. [TNFRSF6B, MUC1, MOK and CXCL8] and/or [IL2RB, SFTPD, TNFRSF1B and AGPT2].

13. Use of the kit, according to claim 12, for the prognosis of patients suffering from sepsis.

14. Use of the kit, according to claim 13, for prognosing whether a patient suffering from sepsis will develop ARDS.

15. Use of the kit, according to any of the claims 13 or 14, wherein the patient suffering from sepsis is a COVID-19 patient.
